# EUROPEAN PATENT APPLICATION

(11) **EP 1 932 545 A1**
(43) Date of publication of application: **18.06.2008**
(21) Application number: 06077195.3
(22) Date of filing: 07.12.2006
(51) Int. Cl.: A61L 9/00, A61L 9/04, A61L 9/12, B60H 3/00

(54) **Diffuser of volatile substances impregnated in solid or semisolid materials**

(71) Applicant: Cipolla, Fiorenzo, 20020 Cogliate (Milano) (IT)
(72) Inventor: Cipolla, Fiorenzo, 20020 Cogliate (Milano) (IT)
(74) Representative: Riccardi, Sergio

(57) **Abstract**

A container made of wood or plastic is disclosed, to diffuse in the environment volatile substances such as fragrances, essences, desodorants, insecticides which were previously impregnated and/or absorbed in solid or semisolid materials like granules of porous materials or permeable gelatines. The container may be used in any environment and more particularly inside a motor vehicle, and is preferably provided with means to hang or fix it in a proper position for diffusion of the substance. The container may take the form of a small bottle with relevant stopper or little box.

## Description

The present invention relates to a container adapted to diffuse volatile substances that were previously embedded and/or absorbed in solid or semisolid materials which the container is filled of.

Various system to diffuse fragrances, desodorants, insecticides, essences and other volatile substances of this kind are long known.

A first category of systems concerns use of heat, by which the volatile substance is delivered when the container or the material containing said substance is heated, but these systems are obviously bound by the need of a source of heat involving energy consumption or of burning materials with consequent smoke emission and/or production of burnt residues, and in any case a manual intervention is required to start delivery of the volatile substance.

For these reasons a second category of systems became established, wherein the volatile substance is cold delivered by simple release in the air. This category comprises for instance phials or small bottles containing volatile liquids, generally provided with a jacket of a material that can be impregnated with the volatile liquid, said bottle being possibly adapted to be suspended inside a motor vehicle. This category comprises also objects of a porous or foamy material absorbing odours and/or delivering fragrances, desodorants, insecticides and the like, that can also be hung inside a motor vehicle, cupboard, wardrobe or any other room. However these systems require disposal of the exhausted articles without possibility to refill them for a further use.

A third category of systems, also cold releasing the volatile substance, consists of the so called pot-pourri, namely jars or bowls containing a mixture of flowers, sweet-smelling plants and scents or a pack of natural or artificial porous stones generally impregnated with essences.

Finally ashtrays are also known, containing natural or artificial powders extinguishing cigarettes and absorbing their smoke. However the systems of this third category are not suitable to be hung in use more particularly inside a moving vehicle.

As far as Applicant knows, nobody tried to use little pieces or granules of said natural or artificial porous stones or suitable semisolid materials filled in a container adapted to be hung or fixed inside a motor vehicle.

Moreover it does not appear that natural or artificial pumice stone was ever used for this purpose. It is known that pumice stone is a porous type of effusive rock containing many little cavities, used as a powder or small pieces to clean articles, as an ingredient for soaps and abrasives and insulating material to make bricks. There is also the artificial pumice produced by slags of iron and steel works, which are molten, solidified and ground into granules. In view of its porosity, pumice resulted to be very suitable to be impregnated with fragrances, essences, scents, desodorants, insecticides and the like an/or to absorb odour and smoke from the environment. A similar result can be obtained with some semisolid scented gelatines.

Thus the present invention relates to a container in which a charge of pumice or gelatine granules impregnated with said volatile substances is filled, said container having a plurality of openings with such shape and size as to allow release of the volatile substance but to prevent escape of granules. The container may be made either of wood, preferably soft wood like beech, which is well suitable for its absorbing and impregnating properties, or of plastics possibly scented with the same aroma of the filled granules. Therefore the product consists of and contains only environment friendly materials, can be easily arranged inside a motor vehicle, for instance by hanging the container to the rear-view mirror through a cord or fixing it to the air vent by a suitable hook or peg.

Moreover this category of containers, unlike those containing the conventional liquid essences, avoids the problem of possible dripping, often causing indelible stains for instance on the dashboard of motor vehicles.

The container may take several forms. A first embodiment consists of a small bottle preferably sealed by a cap or stopper but any other kind of closure may be used. The hanging cord is generally threaded in a ring applied at the stopper top. This container is also adapted to be re-used permanently, as refilling bags of pumice or gelatine granules may be provided, to be placed in the container when the original charge is exhausted, the filled quantity of volatile substance having been used up.

Another possible embodiment of the container consists of a plastic little box containing the natural stones, provided with a peg to be fixed to an air vent iside a motor vehicle and a revolving knob to adjust the intensity of essence release.

Both these preferred embodiments of the container according to the present invention are described as non limiting explanatory examples only with reference to the accompanying sheets of illustrative drawings, in which:
Fig. 1A is a perspective view of the first embodiment of container according to the present invention in the form of small bottle with relevant stopper;
Fig. 1B is a perspective view similar to Fig.1A but with a portion of the container broken to show the contents of impregnated granules; and
Figs. 2A, 2B, 2C are a front view, a side view and a rear view, respectively, of the second embodiment of the container according to the present invention in the form of a little box to be applied with a peg to the air vent of a motor vehicle.

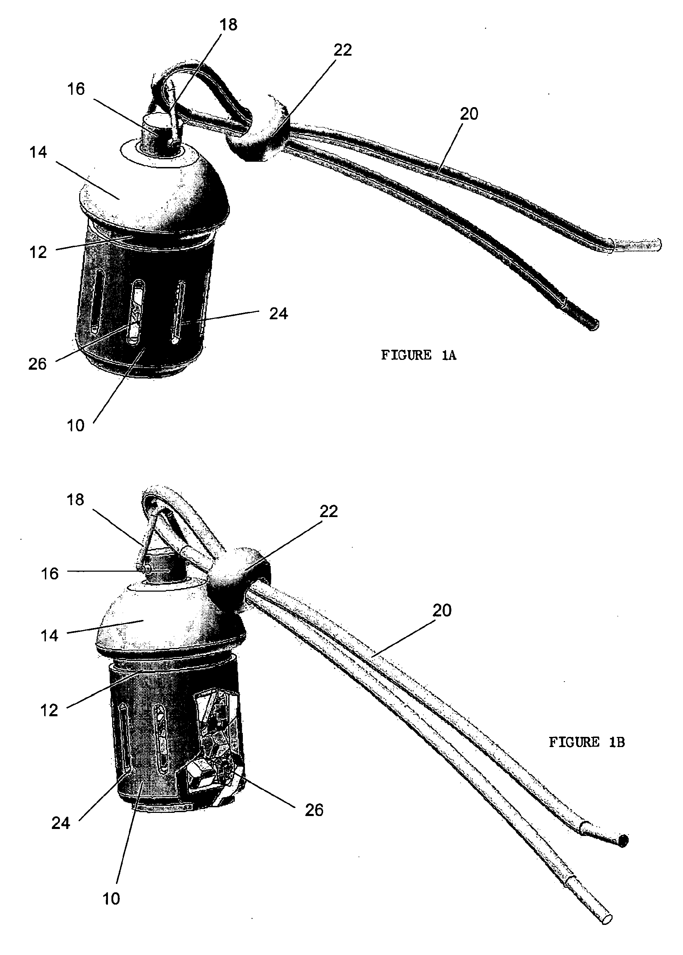

With reference now to Figs. 1A-1B the container of the first embodiment consists of a cylindrical body 10 in the form of small bottle, on whose neck 12 a stopper 14 is forcedly introduced, said stopper having a head 16 in which a ring 18 is applied. A hanging cord 20 is passing through said ring 18 and is preferably provided with a sliding clip 22.

The cylindrical body 10 is provided with a plurality of slots 24 of such shape and size as to allow release of the volatile substance but to hinder escape of pumice or gelatine granules 26 contained inside the cylindrical body 10.

Finally instead of cord 20 a hook or clip may be provided for attachment to the air vent of a vehicle or any other fixing device suitable for the kind of anticipated application.

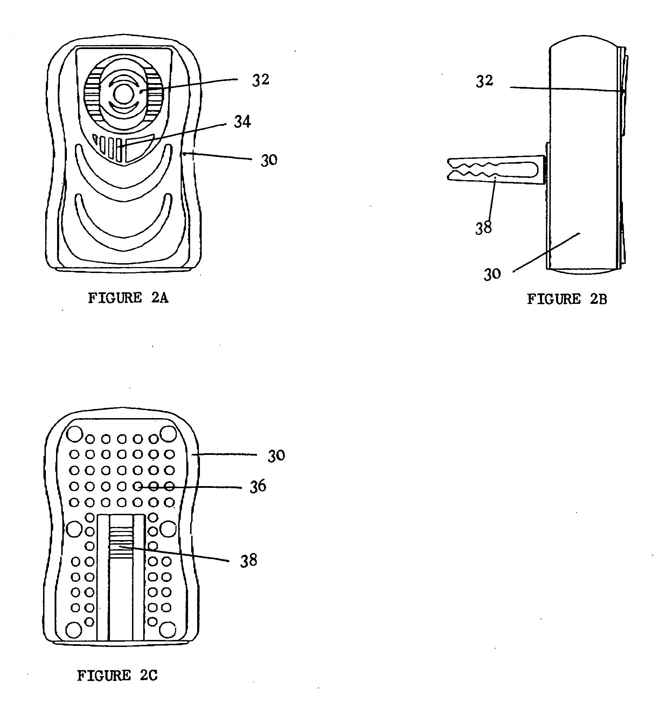

Turning now to illustrate the embodiment shown in Figs. 2A, 2B, 2C, the container consists of a little box 30 preferably made of plastic, properly shaped for a comfortable handling, filled with the natural scented stones, on whose front side there is a hand actuated knob 32 so as to rotate back and forth thus adjusting the intensity of essence release. Below said knob 32 on the front side of the box 30 there is a set of markings 34 indicating to the user the direction of rotation to increase or decrease the intensity of essence release. On the rear side of the box 30 there is a plurality of little holes 36 for essence delivery and the peg 38 is mounted, to fix the box 30 to the grid of the air vent of the vehicle, generally arranged on the dashboard.

Obviously many modifications, additions, substitutions may be made to the container of the invention, without departing however from its general innovative principle nor from its scope of protection, as defined in the appended claims.

## Claims

1. A container for diffusing volatile substances impregnated in solid or semisolid materials, **characterized in that** said container is filled with a charge of granules of said materials and is provided with openings of such a shape and size as to allow release of the volatile substance but to hinder escape of the filled granules.

2. The container of claim 1, **characterized by** comprising a small bottle provided with a stopper sealed in the neck of said bottle.

3. The container of claim 2, **characterized in that** the stopper is provided with a ring in which a cord is threaded to hang the container inside an enclosed space.

4. The container of claim 3, **characterized by** being hung inside a vehicle.

5. The container of claim 4, **characterized by** being suspended on the rear-view mirror of the vehicle.

6. The container of claim 1, **characterized by** comprising a little box provided with a device for its application to a part inside a vehicle.

7. The container of claim 6, **characterized in that** said device is a hook or peg for application to an air vent of the vehicle.

8. The container of claim 6, **characterized in that** said box is provided with a revolving knob to adjust the intensity of release of the volatile substance.

9. The container according to one or more of the preceding claims, **characterized in that** said granules are of natural or artificial pumice stone.

10. The container according to one or more of claims 1-8, **characterized in that** the semisolid material is gelatine.

11. The container according to one or more of the preceding claims, **characterized by** being made of wood, preferably soft wood such as beech.

12. The container according to one or more of claims 1-10, **characterized by** being made of plastic, possibly impregnated with the same volatile substance of the granules therein contained.

13. The container according to one or more of the preceding claims, **characterized in that** the volatile substance is one selected from the group comprising fragrances, essences, scents, desodorants, insecticides and the like.

14. The container according to one or more of the preceding claims, **characterized in that** the granules of solid or semisolid material are also adapted to absorb odours and smoke from the environment.
